# EUROPEAN PATENT APPLICATION

(11) **EP 1 752 531 A1**
(43) Date of publication of application: **14.02.2007**
(21) Application number: 06006808.7
(22) Date of filing: 30.03.2006
(51) Int. Cl.: C12N 9/04, G01N 33/66

(54) **Method for improving substrate specificity of pyrroloquinoline quinone dependent glucose dehydrogenase**

(30) Priority: 11.08.2005 JP 2005233049
(71) Applicant: Toyo Boseki Kabushiki Kaisha, Osaka-shi, Osaka-fu (JP)
(72) Inventor: Kitabayashi, Masao, c/o Toyo Boseki K.K., Tsuruga-shi, Fukui-ken (JP); Aiba, Hiroshi, c/o Toyo Boseki K.K., Tsuruga-shi, Fukui-ken (JP)
(74) Representative: Vossius & Partner

(57) **Abstract**

The present invention relates to a method for reducing activity for maltose in glucose measurement which comprises a step of reacting pyrroloquinoline quinone-dependent glucose dehydrogenase, the method comprising a step of keeping the pH acidic during the measurement reaction.

## Description

The present invention relates to a method for reducing activity for maltose in glucose measurement comprising the step of reacting pyrroloquinoline quinine dependent glucose dehydrogenase (hereinafter, pyrroloquinoline quinine is referred to as "PQQ", glucose dehydrogenase is referred to as "GDH", and pyrroloquinoline quinone dependent glucose dehydrogenase is referred to as "PQQGDH", respectively), a glucose measurement composition wherein the activity for maltose is reduced, a glucose sensor and methods for production thereof.

PQQGDH is glucose dehydrogenase using pyrroloquinoline quinone as a coenzyme, and can be used for assay of blood glucose because it catalyzes a reaction in which glucose is oxidized to produce gluconolactone.

A glucose concentration in blood is a very important indicator as an important marker for diabetes in clinical diagnosis. At present, the glucose concentration in blood is primarily measured by a biosensor.

PQQGDH has been noticed as an enzyme to determine blood glucose level. The inventors have found that *Acinetobacter baumannii* NCIMB11517 strain produces pyrroloquinoline quinine dependent glucose dehydrogenase, cloned a gene thereof and constructed a high expression system thereof, which is disclosed in JP HEI-11-243949 A Publication.

PQQGDH catalyzes a reaction in which D-glucose is oxidized to produce D-glucono-1,5-lactone. PQQGDH is not influenced by dissolved oxygen, and has an enzymatic property of no coenzyme requirement. PQQGDH is expected to apply a variety of applications such as biological diagnostics to assay blood glucose level, and blood glucose sensor. It is also noted that PQQGDH has problems such as acting on disaccharides, in particular, maltose.

The inventors made an extensive research on the cause of the problems to be solved and found that PQQGDH had a low reactivity on ferricyanide ion usually used as a mediator in blood glucose sensor.

The inventors further investigated and demonstrated that the low reactivity on ferricyanide ion was caused by the influence of near neutral buffer conditions leading to a substrate specificity of enzymatic reaction.

WO03/106668 discloses a means to improve the substrate specificity of PQQGDH, wherein PQQGDH gene is modified. However, WO03/106668 does not disclose or suggest a means to solve the problem of substrate specificity from viewpoint of assay conditions.

The inventors searched for a simple approach to improve the substrate specificity and demonstrated that the substrate specificity of PQQGDH can be improved by setting acidic pH for a glucose assay composition, thus accomplished the invention

The invention provides the following Items 1-17.
[Item 1] A method for lowering the activity for maltose in glucose measurement which comprises a step of reacting pyrroloquinoline quinone-dependent glucose dehydrogenase, the method comprising a step of keeping the acidic pH during the measurement reaction.
[Item 2] The method according to Claim 1, wherein the step of reacting pyrroloquinoline quinone-dependent glucose dehydrogenase is accomplished using a glucose measurement reagent composition comprising pyrroloquinoline quinone-dependent glucose dehydrogenase.
[Item 3] The method according to Claim 1, wherein said composition further comprises a mediator.
[Item 4] The method according to Claim 3, wherein the mediator is a ferricyanide salt.
[Item 5] The method according to Claim 1, wherein the pyrroloquinoline quinone-dependent glucose dehydrogenase is modified pyrroloquinoline quinone-dependent glucose dehydrogenase the maltose activity of which is lowered below that of the corresponding wild-type enzyme.
[Item 6] The method according to Claim 1, wherein the pH is an acidic pH of 6.5 or less during the measurement reaction.
[Item 7] The method according to Claim 6, wherein the glucose measurement reagent composition is in a form comprising ferricyanide ions as the mediator.
[Item 8] A method for lowering the activity for maltose in glucose measurement comprising a step of reacting the pyrroloquinoline quinone-dependent glucose dehydrogenase according to Claim 6, wherein the glucose measurement composition forms part of a glucose assay kit.
[Item 9] The method according to Claim 8, wherein the glucose assay kit comprises pyrroloquinoline quinone-dependent glucose dehydrogenase, and the pH is an acidic pH of 6.5 or less during the measurement reaction.
[Item 10] The method according to Claim 1, wherein the step of reacting the pyrroloquinoline quinone-dependent glucose dehydrogenase is accomplished in a glucose sensor including pyrroloquinoline quinone-dependent glucose dehydrogenase and also including electrodes comprising at least a working electrode and a counter electrode.
[Item 11] The method according to Claim 10, wherein the reaction in the glucose sensor comprises applying voltage to a reaction solution comprising pyrroloquinoline quinone-dependent glucose dehydrogenase, and measuring the oxidation current of a mediator.
[Item 12] The method according to Claim 10, wherein the glucose sensor comprises pyrroloquinoline quinone-dependent glucose dehydrogenase, and wherein the pH is an acidic pH of 6.5 or less during the measurement reaction.
[Item 13] The method according to Claim 10, wherein the glucose sensor comprises ferricyanide ions as the mediator.
[Item 14] A glucose measurement composition, comprising modified pyrroloquinoline quinone-dependent glucose dehydrogenase having a modification in the amino acid sequence and a buffer capable of maintaining an acidic pH during the measurement reaction, wherein the activity for maltose is reduced.
[Item 15] A glucose sensor comprising modified pyrroloquinoline quinone-dependent glucose dehydrogenase having mutations in the amino acid sequence and a buffer capable of maintaining an acidic pH during the measurement reaction, wherein the activity for maltose is reduced.
[Item 16] A method for manufacturing a glucose measurement composition which uses pyrroloquinoline quinone-dependent glucose dehydrogenase and in which the activity for maltose is reduced, the method comprising a step of including a buffer capable of maintaining an acidic pH during the measurement reaction.
[Item 17] A method for manufacturing a glucose sensor which uses pyrroloquinoline quinone-dependent glucose dehydrogenase and in which the activity for maltose is reduced, the method comprising a step of including a buffer capable of maintaining an acidic pH during the measurement reaction.

In the present invention, improvement of the substrate specificity of PQQGDH enables improving the accuracy of measuring glucose concentration using a glucose assay reagent, a glucose assay kit, and a glucose sensor.

Figure 1 shows the relationship between substrate concentration and PQQGDH reaction speed in PIPES (pH 5.5) with wild-type PQQGDH;

Figure 2 shows the relationship between substrate concentration and PQQGDH reaction speed in PIPES (pH 6.5) with wild-type PQQGDH;

Figure 3 shows the relationship between substrate concentration and PQQGDH reaction speed in MES (pH 5.5) with wild-type PQQGDH;

Figure 4 shows the relationship between substrate concentration and PQQGDH reaction speed in MES (pH 6.5) with wild-type PQQGDH;

Figure 5 shows the relationship between substrate concentration and PQQGDH reaction speed in phthalic acid (pH 5.5) with wild-type PQQGDH;

Figure 6 shows the relationship between substrate concentration and PQQGDH reaction speed in phthalic acid (pH 6.5) with wild-type PQQGDH;

Figure 7 shows the relationship between substrate concentration and PQQGDH reaction speed in citraconic acid (pH 5.5) with wild-type PQQGDH;

Figure 8 shows the relationship between substrate concentration and PQQGDH reaction speed in citraconic acid (pH 6.5) with wild-type PQQGDH;

Figure 9 shows the relationship between substrate concentration and PQQGDH reaction speed in glutaric acid (pH 5.5) with wild-type PQQGDH;

Figure 10 shows the relationship between substrate concentration and PQQGDH reaction speed in glutaric acid (pH 6.5) with wild-type PQQGDH;

Figure 11 shows the relationship between substrate concentration and PQQGDH reaction speed in PIPES (pH 5.5) with modified PQQGDH A;

Figure 12 shows the relationship between substrate concentration and PQQGDH reaction speed in PIPES (pH 6.5) with modified PQQGDH A;

Figure 13 shows the relationship between substrate concentration and PQQGDH reaction speed in MES (pH 5.5) with modified PQQGDH A;

Figure 14 shows the relationship between substrate concentration and PQQGDH reaction speed in MES (pH 6.5) with modified PQQGDH A;

Figure 15 shows the relationship between substrate concentration and PQQGDH reaction speed in phthalic acid (pH 5.5) with modified PQQGDH A;

Figure 16 shows the relationship between substrate concentration and PQQGDH reaction speed in phthalic acid (pH 6.5) with modified PQQGDH A;

Figure 17 shows the relationship between substrate concentration and PQQGDH reaction speed in citraconic acid (pH 5.5) with modified PQQGDH A;

Figure 18 shows the relationship between substrate concentration and PQQGDH reaction speed in citraconic acid (pH 6.5) with modified PQQGDH A;

Figure 19 shows the relationship between substrate concentration and PQQGDH reaction speed in glutaric acid (pH 5.5) with modified PQQGDH A;

Figure 20 shows the relationship between substrate concentration and PQQGDH reaction speed in glutaric acid (pH 6.5) with modified PQQGDH A;

Figure 21 shows the relationship between substrate concentration and PQQGDH reaction speed in PIPES (pH 5.5) with modified PQQGDH B;

Figure 22 shows the relationship between substrate concentration and PQQGDH reaction speed in PIPES (pH 6.5) with modified PQQGDH B;

Figure 23 shows the relationship between substrate concentration and PQQGDH reaction speed in MES (pH 5.5) with modified PQQGDH B;

Figure 24 shows the relationship between substrate concentration and PQQGDH reaction speed in MES (pH 6.5) with modified PQQGDH B;

Figure 25 shows the relationship between substrate concentration and PQQGDH reaction speed in phthalic acid (pH 5.5) with modified PQQGDH B;

Figure 26 shows the relationship between substrate concentration and PQQGDH reaction speed in phthalic acid (pH 6.5) with modified PQQGDH B;

Figure 27 shows the relationship between substrate concentration and PQQGDH reaction speed in citraconic acid (pH 5.5) with modified PQQGDH B;

Figure 28 shows the relationship between substrate concentration and PQQGDH reaction speed in citraconic acid (pH 6.5) with modified PQQGDH B;

Figure 29 shows the relationship between substrate concentration and PQQGDH reaction speed in glutaric acid (pH 5.5) with modified PQQGDH B; and

Figure 30 shows the relationship between substrate concentration and PQQGDH reaction speed in glutaric acid (pH 6.5) with modified PQQGDH B.

The present invention will be described below in detail.

In the present invention, activity for maltose means the action of PQQGDH dehydrogenating that sugar substrate.

In addition to maltose, activity for at least one other sugar other than glucose selected from the sugars other than maltose can also be reduced.

Sugar substrates other than glucose selected from the sugars other than maltose include for example galactose, mannose, xylose and other simple sugars, sucrose, lactose, cellobiose and other disaccharides, maltotriose, maltotetraose and other oligosaccharides, icodextrin (glucose polymer) and other polysaccharides (an oligosaccharide comprises 2 to 10 molecules of a single sugar while a polysaccharide comprises 11 or more molecules of a single sugar bound together by glycoside bonds or the like, with no restrictions on the binding mode, and in the case of a disaccharide or higher sugar the structure may be homogenous or heterogeneous), as well as the sugar alcohols, 2-deoxy-D-glucose, 3-o-methyl-D-glucose and the like and derivatives of these.

Of these, it is preferably to select various sugars which may pose a problem when the PQQGDH of the present invention is used for clinical diagnosis or controlling the blood glucose levels of diabetes patients, or for measuring blood glucose concentrations. Examples of such sugars include mannose, allose, xylose, galactose, maltose and the like, and more desirable examples are galactose and lactose. Maltose is the most desirable example.

The following method is used to judge whether activity for a specific sugar has been reduced.

In the activity measurement method described in Test Example 1 below, using PQQGDH, the PQQGDH activity value (a) using D-glucose as the substrate solution and the PQQGDH activity value (b) using this sugar in place of D-glucose as the substrate solution are measured, and the relative value ((b)/(a) x 100) is calculated given 100 as the measurement value using glucose as the substrate. The same operation is then repeated with the conditions changed, and the values are compared and evaluated.

Activity is measured by the activity measurement method described in Test Example 1 below.

The PQQGDH that can be used in the method of the present invention is an enzyme (EC 1.1.5.2 (old EC 1.1.99.17)) having pyrroloquinoline quinone as the coenzyme which catalyzes a reaction in which D-glucose is oxidized to produce D-glucono-1,5-lactone, with no particular limits on its derivation or structure.

An origin of PQQGDH to be used for the present invention is not specifically limited. Representative origins of the wild type PQQGDH which is the source of the modification are microorganisms exemplified below. Specifically, examples may include oxidizing bacteria such as *Acinetobacter baumannii(*JP HEI-11-243949 A), *Acinetobacter calcoaceticus* (eg. A.M.Cleton-Jansen et al J. Bacteriol., 170, 2121 (1988); and Mol.Gen.Genet., 217, 430 (1989)), *Pseudomonas aeruginosa, Pseudomonas putida, Pseudomonas fluorescens* and *Gluconobacter oxydans,* and enterobacteria such as *Agrobacterium radiobacter, Escherichia coli* (A.M.Cleton-Jansen et al J. Bacteriol., 172, 6308(1990)) and *Klebsiella aerogenes, Burkhorderia cepacia.*
It is preferable to select those derived from the microorganisms belonging to the genus *Acinetobacter* as the origin. These are water-soluble enzymes and easily dissolved in an aqueous system. More preferably, it is preferable to select the soluble PQQGDH from *Acinetobacter calcoaceticus* or *Acinetobacter baumannii.* Particularly preferable PQQGDHs are derived from *Acinetobacter baumannii* NCIMB 11517 strain (see, JP HEI-11-243949 A), *Acinetobacter calcoaceticus* LMD79.41 strain (see, A.M.Cleton-Jansen et al J. Bacteriol., 170, 2121 (1988); and Mol.Gen.Genet., 217, 430 (1989)), and *Acinetobacter calcoaceticus* IFO 12552 strain (see, JP 2004-173538 A). Most preferable are PQQGDH derived from *Acinetobacter* baumannii NCIMB 11517 strain. The *Acinetobacter baumannii* NCIMB11517 strain was previously classified into *Acinetobacter calcoaceticus.*

As long is it has glucose dehydrogenase activity, the PQQGDH that can be used in the method of the present invention may have some of the other amino acid residues deleted or substituted or other amino acid residues added in those sequences given as examples above.
Such modification can be easily performed by the skilled artisan according to known techniques in the art. A variety of methods for introducing a site-directed mutagenesis to a protein by substituting or inserting one or more bases to a nucleotide sequence of a gene coding for the protein are disclosed in Sambrooket al, Molecular Cloning; A Laboratory Manual 2nd Eddition(1989)Cold Spring Harbor Laboratory Press,New York.

Toyo Boseki GLD-321 and other commercial products can be used for these PQQGDH enzymes. Alternatively, they can be easily manufactured by a person skilled in the art using known techniques in the field.

For example, naturally-occurring microorganisms producing the PQQGDH, or transformant prepared by inserting a naturally-occurring or modified PQQGDH gene into an expression vector (a variety of vectors including a plasmid are known), followed by transforming a suitable host (a variety of hosts including E. coli are known) with the expression vector, are cultured, host cells are collected from a culture medium by centrifugation, cells are broken down mechanically or enzymatically with lysozyme, optionally solubilized by the addition of a chelating agent such as EDTA or a surfactant to obtain a water soluble fraction. The expressed PQQGDH can be secreted to a culture medium using a suitable host-vector system.

PQQGDH can be separated and precipitated from the PQQGDH-containing solution by concentration under reduced pressure, membrane concentration, salting out using ammonium sulfate or sodium sulfate, or a fractional precipitation with a hydrophilic solvent such as methanol, ethanol, acetone, etc. Heat treatment and isoelectric treatment are also an effective purification method. Purified PQQGDH can be obtained by gel filtration with adsorbent or gel filtering agent, adsorption chromatography or affinity chromatography. The standard enzyme is preferably purified enough to show a single band in electrophoresis (SDS-PAGE).

The PQQGDH can be heat-treated at 25 to 50°C, preferable 30 to 45°C to increase a proportion of holoenzyme to the total GDH protein before or after the above-mentioned steps.

Concentration of PQQGDH of the invention is not specifically limited.

Enzymatic activity of PQQGDH can be measured according to the following method.

### Method for assaying Enzymatic activity of PQQGDH

### (1) Principle of measurement

D-glucose + PMS + PQQGDH → D-glucono-1,5-lactone + PMS (red) 2PMS (red) + NTB → 2PMS + diformazan

The presence of diformazan formed by reduction of nitrotetrazolium blue (NTB) by phenazine methosulfate (PMS) (red) was measured by spectrophotometry at 570 nm.

### (2) Definition of unit

One unit refers to the amount of the enzyme of PQQGDH to form 0.5 mM of diformazan per one minute under the following condition.

### (3) Method

### Reagent

A. D-Glucose solution: 0.5 M (0.9 g D-glucose, molecular weight: 180.16)/10 mL H₂O
B. PIPES-NaOH buffer pH 6.5: 50 mM (1.51 g of PIPES [molecular weight: 302.36] was suspended in 60 mL of water) was dissolved in 5 N NaOH, and 2.2 mL of 10% Triton X-100 is added. pH was adjusted to 6.5 ± 0.05 at 25°C using 5 N NaOH, and water was added to make 100 mL.)
C. PMS solution: 3.0 mM (9.19 mg of phenazine methosulfate [molecular weight: 817.65])/10 mL H₂O
D. NTB solution: 6.6 mM (53.96 mg of nitrotetrazolium blue [molecular weight: 817.65])/10 mL H₂O
E. Enzyme dilution solution: 50 mM PIPES-NaOH buffer (pH 6.5) containing 1 mM CaCl₂, 0.1% Triton X-100 and 0.1% BSA

### Procedure

The following reaction mixture was prepared in a light shielding bottle, and stored on ice (prepared at use).

1.8 mL of D-glucose solution (A)

24.6 mL of PIPES-NaOH solution (pH 6.5) (B)

2.0 mL of PMS solution (C)

1.0 mL of NTB solution (D)

| Concentration in assay mixture | |
|---|---|
| PIPES buffer | 42 mM |
| D-glucose | 30 mM |
| PMS | 0.20mM |
| NTB | 0.22mM |

The reaction mixture (3.0 mL) was placed in a test tube (made from plastic), which was then preliminarily heated at 37°C for 5 minutes. The enzyme solution (0.1 mL) was added, and mixed by gently inverting.

The increase of absorbance for water at 570 nm was recorded by a spectrophotometer for 4 to 5 minutes with keeping the temperature at 37°C, and ΔOD per minute was calculated from an initial linear part of a curve (OD test).

At the same time, the same method except for adding the enzyme dilution solution (E) in place of the enzyme solution was repeated to measure a blank (ΔOD blank).

The enzyme powder was dissolved in the ice-cooled enzyme dilution solution (E) just before the assay, and diluted with the same buffer to 0.1 to 0.8 U/mL (due to adhesiveness of the enzyme, it is preferable to use the plastic tube). Calculation

The activity is calculated using the following formulae:
U/ml={ΔOD/min(ΔOD test - ΔOD blank)×Vt×df}/(20.1×1.0×Vs)
U/mg=(U/ml)×1/C
Vt: total volume (3.1 mL)
Vs: sample volume (1.0 mL)
20.1: 1/2 mM molecular absorbance coefficient of diformazan
1.0: light path length (cm)
df: dilution factor
C: enzyme concentration in solution (c mg/mL)

In the method of the present invention the pH is acidic during measurement. An acidic pH is a pH of less than 7.0 without any particular limitations, but in the present invention the pH is preferably 6.5 or less or more preferably 3.5 to 5.5.

In the present invention, a variety of buffers can be used in order to provide an acidic pH during measurement. The buffers are not limited to any particular buffer, as long as the buffers have a buffer capacity to maintain pH in acidic range. Conventionally used buffers include Tris-HCl, borate, phosphate, acetate, succinate, phthalate, maleate, glycine or a salt thereof, Good's buffer including MES, Bis-Tris, ADA, PIPES, ACES, MOPSO, BES, MOPS, TES, HEPES and so on.

Buffers without forming salt with calcium are preferable.

Buffers can be used alone or in combination of two or more. Composite buffers containing one or more buffers other than above-mentioned buffers can also be used.

A concentration of the added buffer is not specifically limited, as long as it is within a concentration range with sufficient buffer capacity, but preferably 100mM or less, more preferably 50mM or less, and also 5mM or more.

A buffer content in a lyophilized product is not specifically limited, but preferably 0.1 mass%, particularly preferably 0.1 to 30 mass%.

A variety of buffers are commercially available. The buffer can be added to a reaction mixture when use, or preliminarily included when a glucose assay reagent, a glucose assay kit, or a glucose sensor described later is prepared. The buffer is added in any form such as liquid or dry powder, as long as it acts in measurement conditions.

Improving substrate specificity in the present invention means improving reactivity of the PQQGDH enzyme for glucose or for a sugar other than glucose. Examples of sugars other than glucose include particularly the disaccharides maltose, sucrose, lactose, cellobiose and the like, especially maltose. In the invention of this application, a decrease in activity for a disaccharide is also described as an improvement in substrate specificity.

The following method is used to evaluate whether activity for a disaccharide has been reduced.

In the activity measurement method described in Test Example 1 below, using PQQGDH, the PQQGDH activity value (a) using D-glucose as the substrate solution and the PQQGDH activity value (b) using this disaccharide in place of D-glucose as the substrate solution are measured, and the relative value ((b)/(a) x 100) is calculated given 100 as the measurement value using glucose as the substrate.

The effect of the invention is especially noticeable in a system containing a mediator. The mediators used in the method of the invention are not specifically limited, but include a combination of phenazine methosulfate(PMS) and 2,6-dichlorophenolindophenol (DCPIP), a combination of (PMS) and nitrotetrazolium blue (NTB), DCPIP alone, ferricyanide ion alone (derived from ferricyanide compounds such as potassium ferricyanide), ferrocene alone. Ferricyanide ion derived from ferricyanide compounds such as potassium ferricyanide is preferable.

A concentration of an added mediator is unnecessary to determined in a specific range due to variation of sensitivity of mediators, but is generally at least 1 mM.

The mediator can be added to a reaction mixture when use, or preliminarily included when a glucose assay reagent, a glucose assay kit, or a glucose sensor described later is prepared. The mediator is added in any form such as liquid or dry powder, as long as it acts in measurement conditions.
A variety of components including surfactants, stabilizers, carriers and excipients can be added to the assay system of the invention.

For example, PQQGDH can be stabilized still further by addition of calcium ions or salts thereof and glutamic acid, glutamine, lysine and other amino acids as well as serum albumin and the like.

For example, PQQGDH can be stabilized by the addition of calcium ion or one or more calcium salts. Calcium salts include calcium salts with inorganic acids or organic acids such as calcium chloride, calcium acetate and calcium citrate. Calcium ion content in an aqueous composition is preferably 1x10⁻⁴ to 1x10⁻² M.

The stabilizing effect based on the addition of calcium ion or calcium salt or salts is further improved by the addition of one or more amino acids selected from the group consisting of glutamic acid, glutamine and lysine. Bovine serum albumin (BSA) and/or ovalbumin can also be included. In addition, PQQGDH can be stabilized by a combination of (i) one or more compounds selected from the group consisting of aspartic acid, glutamic acid, α-ketoglutaric acid, malic acid, α-ketogluconic acid and α-cyclodextrin, and (ii) alubumin.

A glucose level can be determined by a variety of methods of the invention. The glucose assay reagent, glucose assay kit, or glucose sensor of the invention can be prepared in a variety of forms including liquid (aqueous solution, suspension), powder (prepared by vacuum dry or spray dry), lyophilized form, etc. Lyophilization is not limited and may be carried out according a conventional method. The composition including enzyme of the invention include a lyophilized product and a solution prepared by redissolving the lyophilized product. Glucose measurement reagent

The glucose measurement reagent of the present invention typically includes PQQGDH and buffers, mediators and other reagents necessary for measurement, a glucose standard solution for preparing the calibration curve, and directions for use. The kit of the present invention can be provided for example as a freeze-dried reagent or as a solution in a suitable storage solution. Preferably the PQQGDH of the present invention is provided in holoenzyme form, but it can also be provided in apoenzyme form and converted to holoenzyme form for use. Glucose assay Kit

The glucose assay kit of the present invention typically includes PQQGDH and buffers, mediators and other reagents necessary for measurement, along with a glucose standard solution for preparing the calibration curve and directions for use. The kit of the present invention can be provided for example as a freeze-dried reagent or as a solution in a suitable storage solution. Preferably the PQQGDH of the present invention is provided in holoenzyme form, but it can also be provided in apoenzyme form and converted to holoenzyme form for use. Glucose sensor

The glucose sensor of the present invention uses a carbon electrode, gold electrode, platinum electrode or the like as the electrode on which PQQGLD is fixed. Fixing methods include a method using a crosslinking reagent, a method of sealing inside a polymer matrix, a method of covering with a transparent film, and methods using photo-crosslinking polymers, conductive polymers, oxidation-reduction polymers and the like, or a method of fixing by adsorption on an electrode or fixing in a polymer together with an electron mediator such as ferrocene or a derivative thereof, or a combination of these methods. The PQQGDH of the present invention is preferably fixed on the electrode as a holoenzyme, but it can also be fixed as an apoenzyme and PQQ supplied in a separate layer or in solution. Typically the PQQGDH of the present invention is first fixed on a carbon electrode using glutaraldehyde, and treated with a reagent having amine groups to block the glutaraldehyde.

Glucose concentration can be measured as described below. Buffer liquid is placed in a thermostatic cell, CaCl₂ is added together with a mediator and the temperature is kept constant. Potassium ferricyanide, phenazine sulfate or the like can be used as the mediator. An electrode having the PQQGDH of the present invention fixed thereon is used as the working electrode, together with a counter electrode (such as a platinum electrode) and a reference electrode (such as an Ag/AgCl electrode). A fixed voltage is applied to the carbon electrode, and once the current has become constant a reagent comprising glucose is added and the increase in current is measured. The glucose concentration in the sample is calculated against a calibration curve using a glucose solution of a standard concentration.

The present invention will be described in detail below based on Examples. However the invention is in no way limited to the examples.

### Example 1

### (1) Confirmation of substrate specificity using glucose assay system

### Principle of measurement

D-glucose + ferricyanide ion + PQQGDH → D-glucono-1,5-lactone + ferrocyanide ion

Specificity to each substrate can be determined by changing D-glucose to other saccharides.
The presence of the ferrocyanide ion produced by reduction of the ferricyanide ion was confirmed by measuring the decrease of absorbance at a wavelength of 420 nm by spectrophotometry.

### (2) Method

### Reagent

A. Buffers: PIPES-NaOH buffer pH 6.5: 50 mM (1.51 g of PIPES [molecular weight: 302.36] was suspended in 60 mL of water) was dissolved in 5 N NaOH, and 2.2 mL of 10% Triton-X100 is added. pH was adjusted to 6.5 ± 0.05 at 25°C using 5 N NaOH, and water was added to make 100 mL.) The various buffers (MES-NaOH buffer (pH 6.5), phthalic acid-NaOH buffer (pH 6.5), citraconic acid-NaOH buffer (pH 6.5) and glutaric acid-NaOH buffer (pH 6.5)) were prepared by the same methods as for PIPES.)
B. Potassium ferricyanide solution: 50 mM (0.165 g potassium ferricyanide (molecular weight 329.25) dissolved in 10 ml of distilled water).
C. PQQGDH solution: 1000 U/mL (about 10 mg of PQQGDH (TOYOBO, GLD-331) was dissolved in 10 mL distilled water)

### Sample

D-Glucose solution: Concentrations of 150, 300, 450, 600, 750, 900, 1050, 1200, 1350, and 1500 mM (prepared by 1/10, 2/10, 3/10, 4/10, 5/10, 6/10, 7/10, 8/10, and 9/10 dilution with water using as the standard a 1500 mM glucose solution prepared from 270 g D-glucose (molecular weight 180.16)/1000 ml H₂O
Maltose solutions: Concentrations of 150, 300, 450, 600, 750, 900, 1050, 1200, 1350 and 1500 mM (prepared by 1/10, 2/10, 3/10, 4/10, 5/10, 6/10, 7/10, 8/10 and 9/10 dilution with water using as the standard a 1500 mM maltose solution prepared from 270 g maltose (molecular weight 180.16)/1000 ml H₂O).

### Procedure

1. The following reaction mixture was prepared in a light shielding bottle, and stored on ice (prepared at use).
   49.6 mL of each buffer solution (pH6.5) (A)
   4.0 mL of potassium ferricyanide solution (B)
   5.6 mL of (C)
   1.0 mL of NTB solution (D)
2. The reaction mixture (3.0 mL) was placed in a test tube (made from plastic), which was then preliminarily heated at 37°C for 5 minutes.
3. The glucose solution (0.1 mL) was added, and gently mixed.
4. The decrease of absorbance for water at 420 nm was recorded by a spectrophotometer for 1 to 3 minutes with keeping the temperature at 37°C, and (OD per minute was calculated from an initial linear part of a curve (OD test). At the same time, the same method except for adding distilled water in place of the glucose solution was repeated to measure a blank (OD blank).

The operation was conducted using each glucose solution at a glucose concentration of 150 mM to 1500 mM.

### Calculation

Variation in absorbance per unit time was determined by calculating ΔOD/min(ΔOD test - ΔOD blank)
A glucose concentration in a reaction solution was plotted in a horizontal axis, and ΔOD/min corresponding to each glucose concentration was plotted in a vertical axis.

To evaluate whether activity had declined with respect to sugars other than glucose, the ΔOD/min (a) with D-glucose as the substrate solution and the ΔOD/min (b) with the other sugar in place of D-glucose as the substrate solution were measured, and the relative value ((b)/(a) x 100) was calculated given 100 as the measurement value with glucose as the substrate.

### Example 2

### Investigation of improvements achieved in substrate specificity by changing the pH conditions in a glucose measurement system

This was done based on the methods used in Example 1, with the pH changed from 6.5 to 5.5.

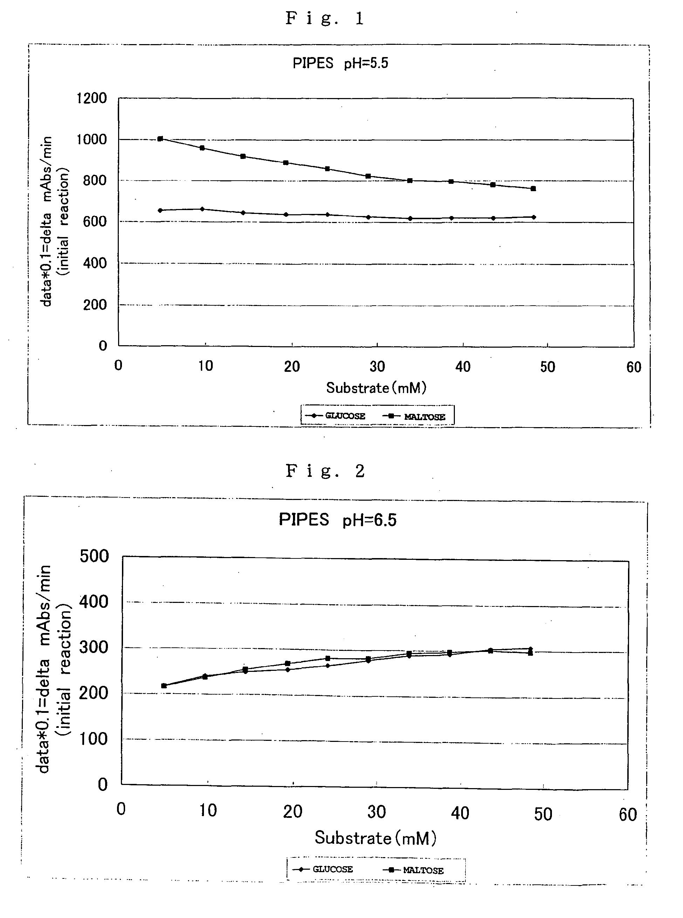

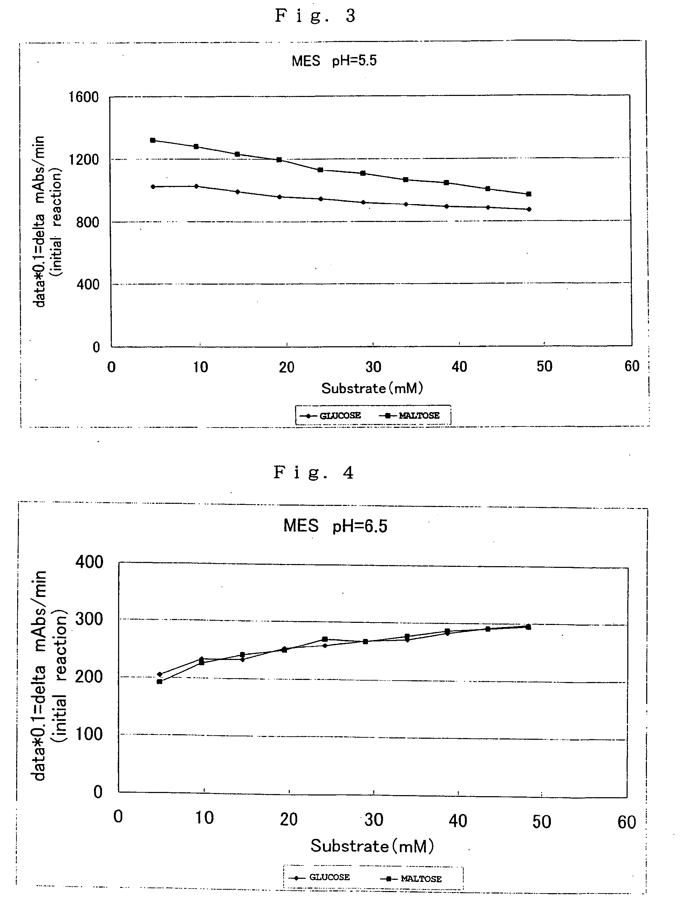

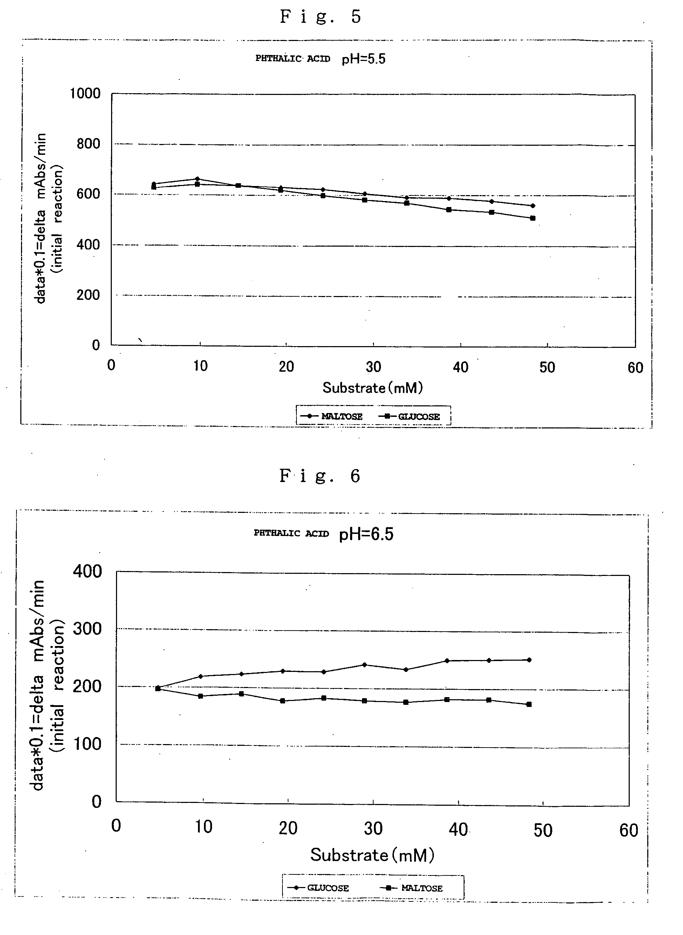

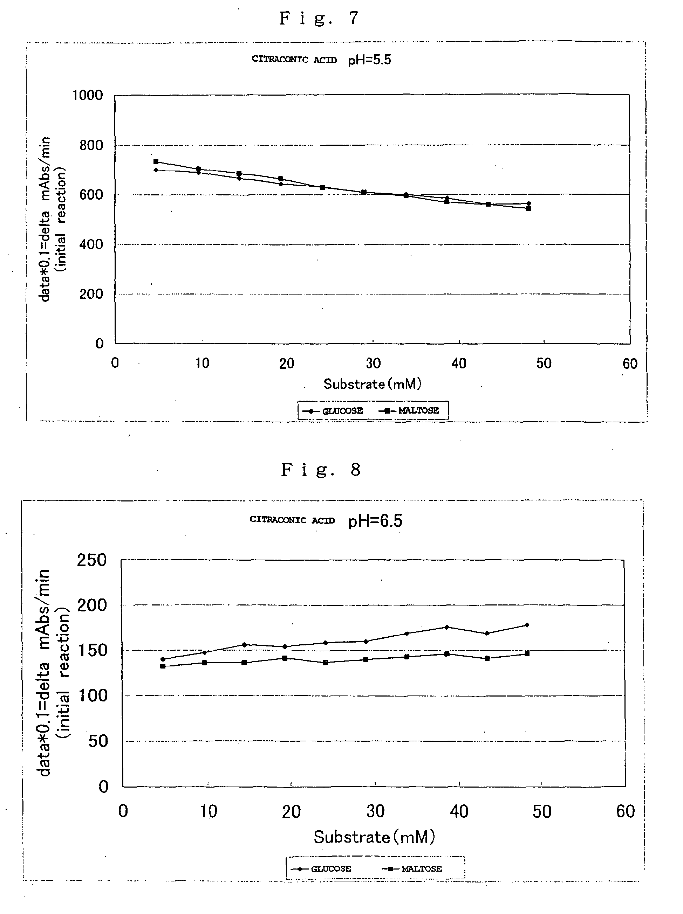

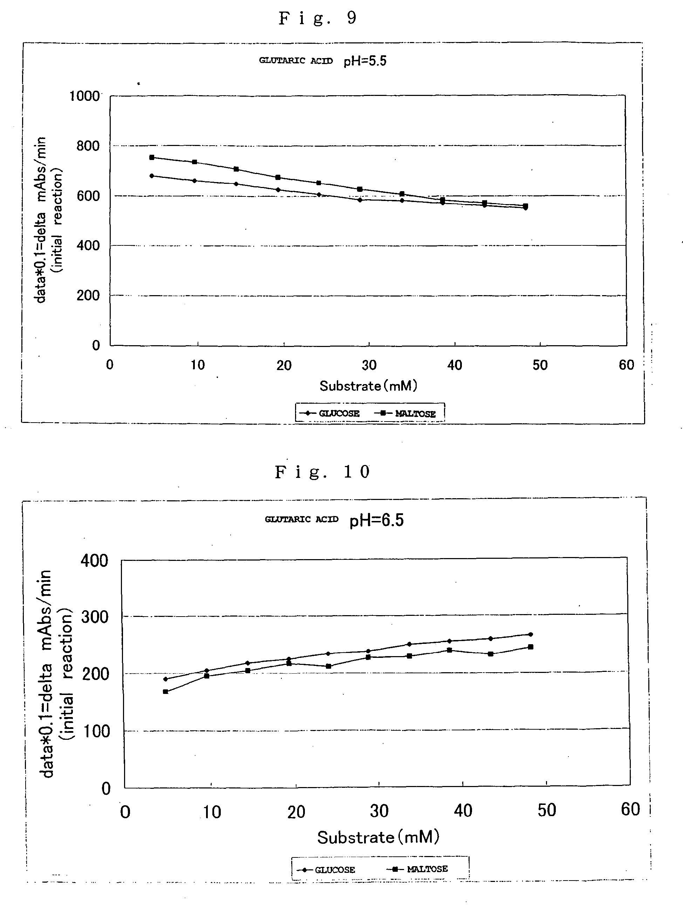

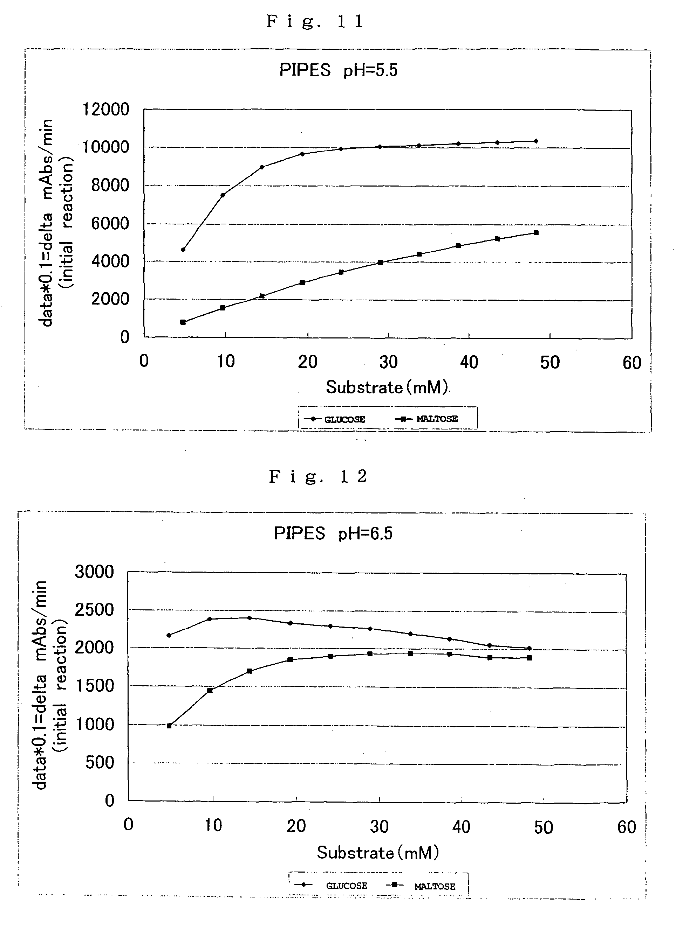

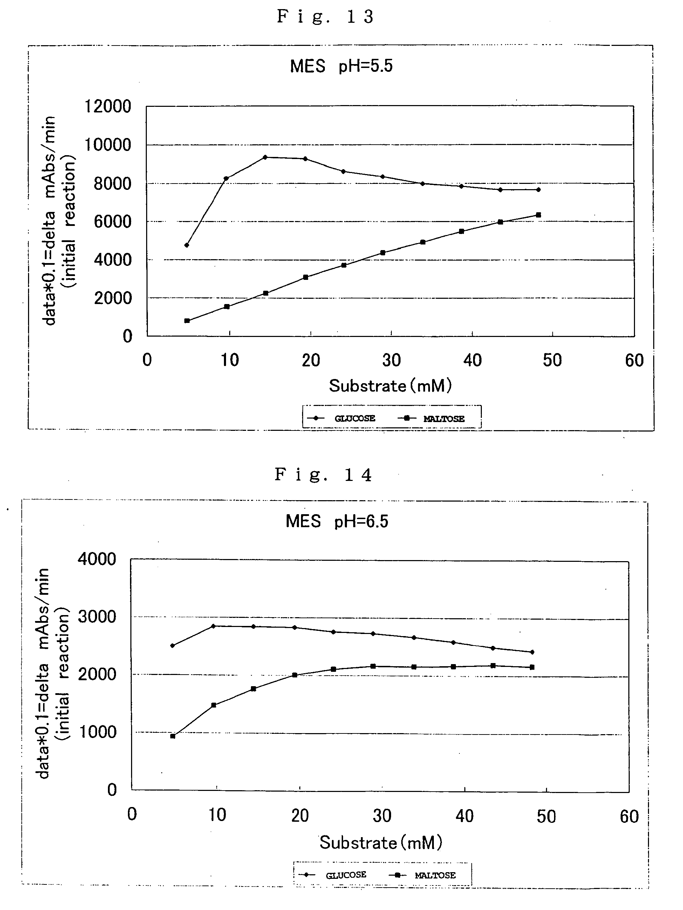

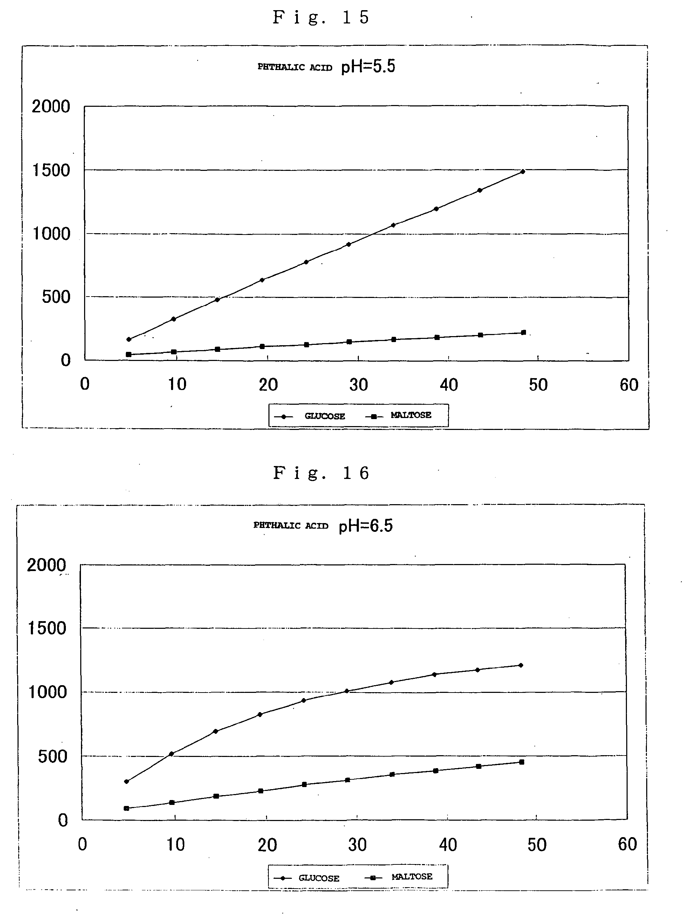

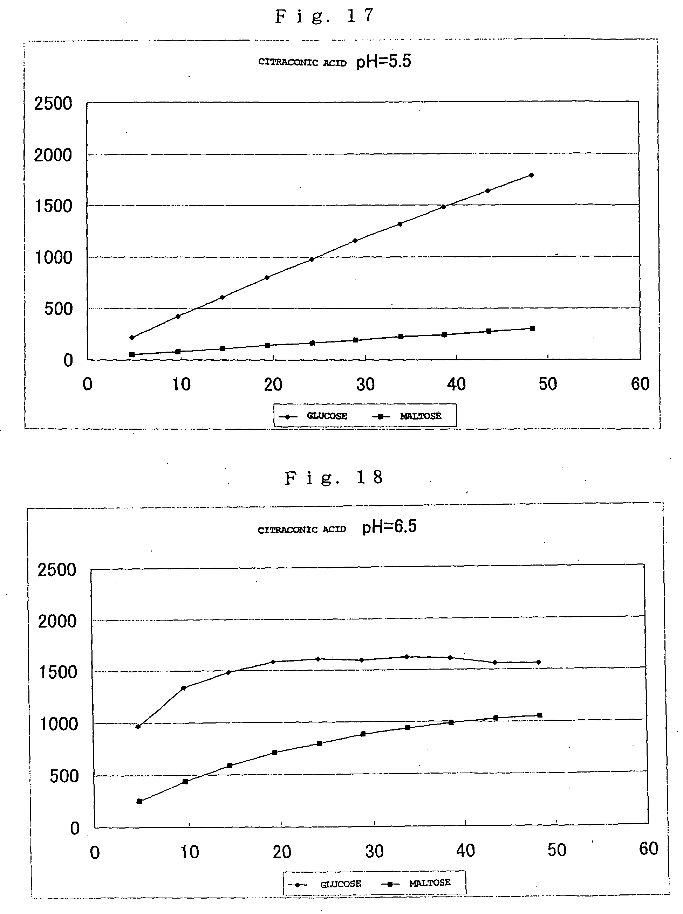

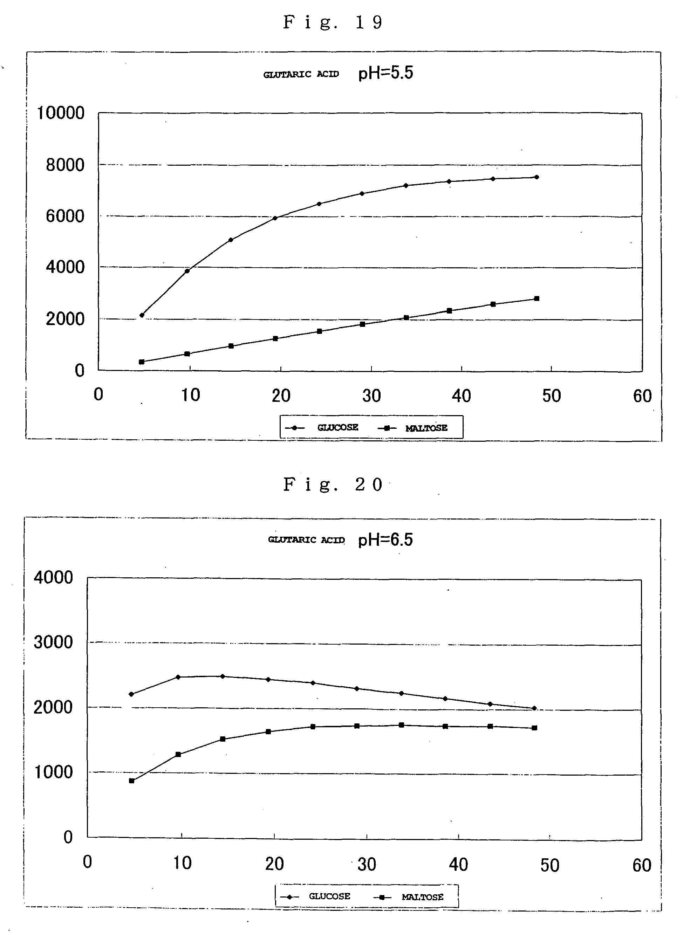

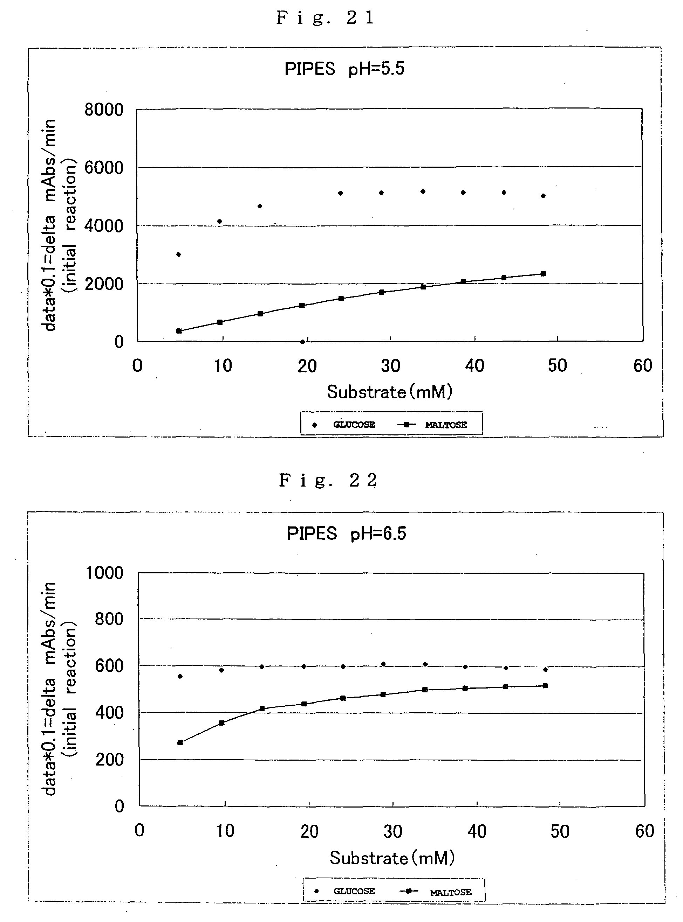

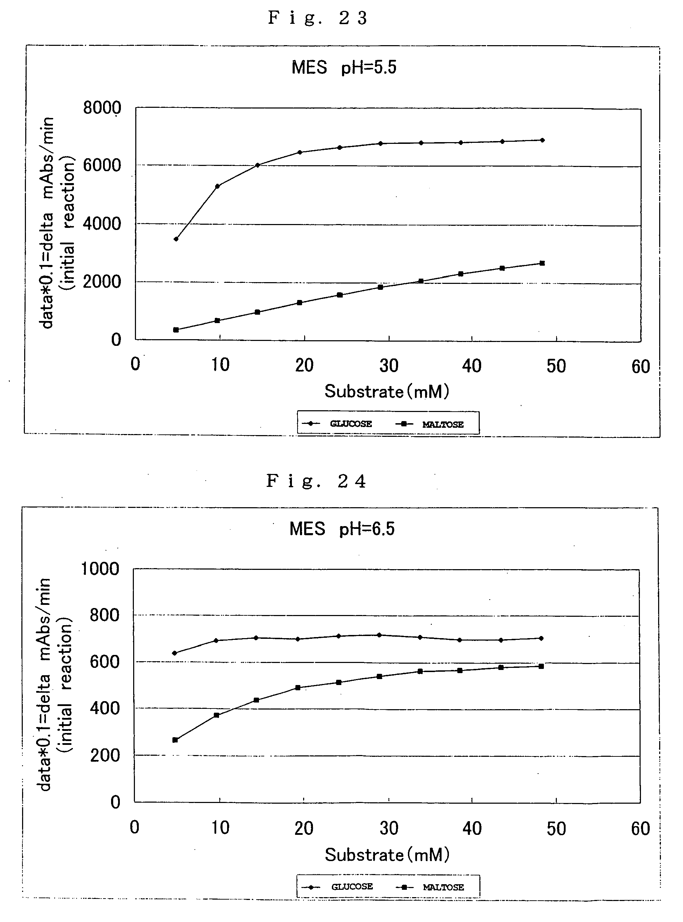

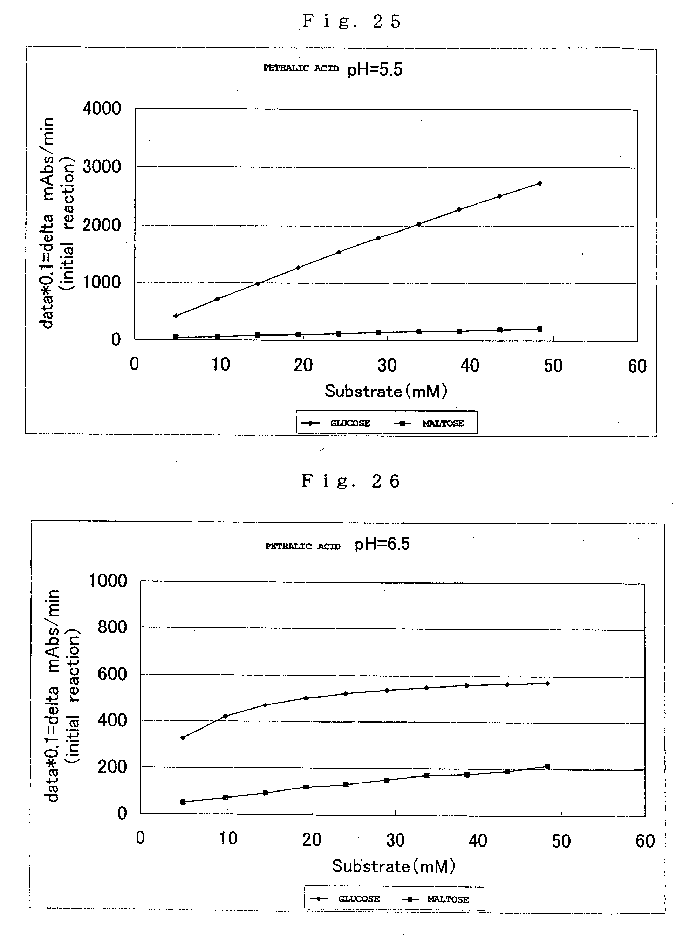

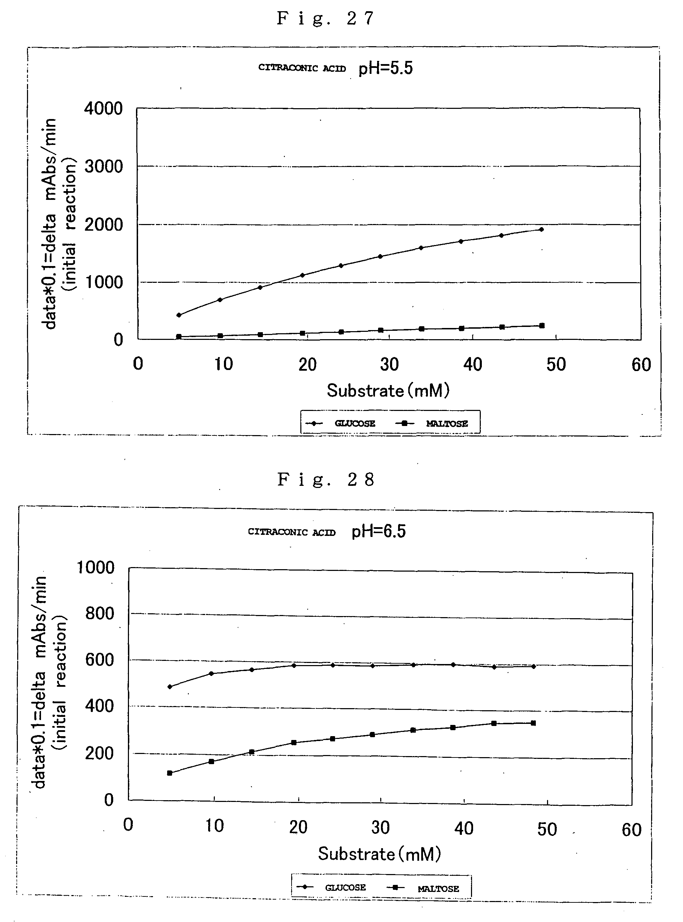

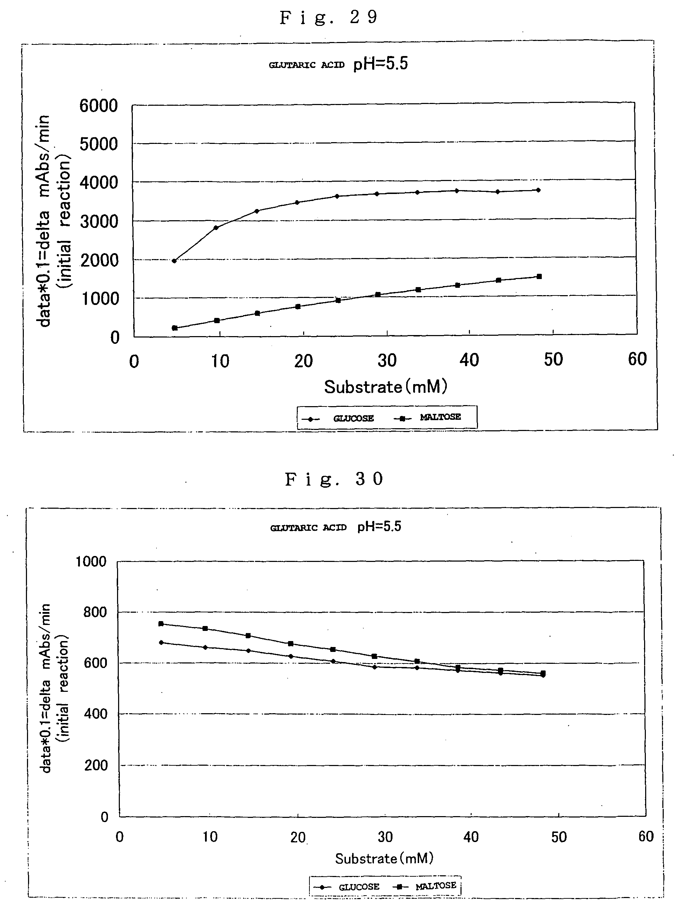

Multiple buffers including Good buffers (PIPES, MES) and acid buffers (phthalic acid, citraconic acid, glutaric acid) were used as the various buffers (A) to confirm that the effects of pH were greater than the effects of buffer composition. The other conditions were all as in Example 1. In Figures 1 through 30, odd numbers indicate results with a buffer of pH 5.5, while even numbers indicate results with a buffer of pH 6.5. Figures 1 through 10 shows the results for wild-type PQQGLD, Figures 11 through 20 shows the results for modified enzyme A with improve substrate specificity, and Figures 21 through 30 shows results for modified enzyme B with improved substrate specificity. Figures 1, 2, 11, 12, 21 and 22 shows results for PIPES buffer, Figures 3, 4, 13, 14, 23 and 24 show results for MES buffer, Figures 5, 6, 15, 16, 25 and 26 shows results for phthalic acid buffer, Figures 7, 8, 17, 18, 27 and 28 show results for citraconic acid buffer, and Figures 9, 10, 19, 20, 29 and 30 show results with glutaric acid buffer.

Comparing the results for wild-type PQQGLD in Figures 1 through 10, it was shown that when the pH of the buffer was lowered specificity of PQQGLD for glucose tended to decline regardless of the buffer composition. Using modified enzymes A and B having improved substrate specificity for glucose, unlike in the case of the wild-type PQQGLD, the specificity of PQQGLD having improved activity for glucose tended to rise when the pH of the buffer was lowered, regardless of the buffer composition. Activity for maltose (%) at each substrate concentration is shown in Tables 1-3.

**Table 1**

| | | mM | **Mal/Glu** |
|---|---|---|---|
| PIPES | pH=5.5 | 4.8 | **153.3%** |
| | | 9.7 | **145.0%** |
| | | 14.5 | **142.6%** |
| | pH=6.5 | 4.8 | **99.1%** |
| | | 9.7 | **98.8%** |
| | | 14.5 | **102.0%** |
| MES | pH=5.5 | 4.8 | **128.8%** |
| | | 9.7 | **124.5%** |
| | | 14.5 | **124.0%** |
| | pH=6.5 | 4.8 | **93.7%** |
| | | 9.7 | **97.0%** |
| | | 14.5 | **103.4%** |
| Phthalic acid | pH=5.5 | 4.8 | **97.5%** |
| | | 9.7 | **96.8%** |
| | | 14.5 | **100.0%** |
| | pH=6.5 | 4.8 | **98.5%** |
| | | 9.7 | **84.0%** |
| | | 14.5 | **84.4%** |
| Citraconic acid | pH=5.5 | 4.8 | **104.7%** |
| | | 9.7 | **102.2%** |
| | | 14.5 | **102.8%** |
| | pH=6.5 | 4.8 | **94.3%** |
| | | 9.7 | **92.5%** |
| | | 14.5 | **87.2%** |
| Glutaric acid | pH=5.5 | 4.8 | **110.7%** |
| | | 9.7 | **111.0%** |
| | | 14.5 | **108.9%** |
| | pH=6.5 | 4.8 | **88.4%** |
| | | 9.7 | **95.1%** |
| | | 14.5 | **94.0%** |

**Table 2**

| | | mM | **Mal/Glu** |
|---|---|---|---|
| PIPES | pH=5.5 | 4.8 | **17.3%** |
| | | 9.7 | **20.5%** |
| | | 14.5 | **24.3%** |
| | pH=6.5 | 4.8 | **45.5%** |
| | | 9.7 | **60.7%** |
| | | 14.5 | **70.7%** |
| MES | pH=5.5 | 4.8 | **16.6%** |
| | | 9.7 | **18.7%** |
| | | 14.5 | **24.1%** |
| | pH=6.5 | 4.8 | **37.2%** |
| | | 9.7 | **51.9%** |
| | | 14.5 | **62.1%** |
| Phthalic acid | pH=5.5 | 4.8 | **27.3%** |
| | | 9.7 | **20.3%** |
| | | 14.5 | **18.2%** |
| | pH=6.5 | 4.8 | **29.8%** |
| | | 9.7 | **26.4%** |
| | | 14.5 | **27.2%** |
| Citraconic acid | pH=5.5 | 4.8 | **23.6%** |
| | | 9.7 | **19.1%** |
| | | 14.5 | **18.0%** |
| | pH=6.5 | 4.8 | **26.3%** |
| | | 9.7 | **32.9%** |
| | | 14.5 | **39.6%** |
| Glutaric acid | pH=5.5 | 4.8 | **15.6%** |
| | | 9.7 | **17.0%** |
| | | 14.5 | **19.1%** |
| | pH=6.5 | 4.8 | **39.5%** |
| | | 9.7 | **52.0%** |
| | | 14.5 | **61.4%** |

**Table 3**

| | | mM | **Mal/Glu** |
|---|---|---|---|
| PIPES | pH=5.5 | 4.8 | **12.1%** |
| | | 9.7 | **16.3%** |
| | | 14.5 | **20.7%** |
| | pH=6.5 | 4.8 | **49.1%** |
| | | 9.7 | **61.3%** |
| | | 14.5 | **70.0%** |
| MES | pH=5.5 | 4.8 | **9.8%** |
| | | 9.7 7 | **12.7%** |
| | | 14.5 | **16.1%** |
| | pH=6.5 | 4.8 | **41.4%** |
| | | 9.7 | **53.7%** |
| | | 14.5 | **62.1%** |
| Phthalic acid | pH=5.5 | 4.8 | **10.2%** |
| | | 9.7 | **7.6%** |
| | | 14.5 | **8.7%** |
| | pH=6.5 | 4.8 | **15.2%** |
| | | 9.7 | **16.7%** |
| | | 14.5 | **19.4%** |
| Citraconic acid | pH=5.5 | 4.8 | **10.7%** |
| | | 9.7 | **9.4%** |
| | | 14.5 | **9.8%** |
| | pH=6.5 | 4.8 | **24.4%** |
| | | 9.7 | **31.1%** |
| | | 14 . 5 | **37.9%** |
| Glutaric acid | pH=5.5 | 4.8 | **11.4%** |
| | | 9.7 | **14.6%** |
| | | 14.5 | **18.3%** |
| | pH=6.5 | 4.8 | **74.2%** |
| | | 9.7 | **81.4%** |
| | | 14.5 | **90.6%** |

Thus, it has been shown that with a modified enzyme with improved substrate specificity for glucose, substrate specificity can be further improved 1.5 to 6.5 times by shifting the pH of the buffer from near neutral to the acidic range.

With the improved substrate specificity achieved with the present invention it is possible to improve the measurement accuracy of glucose measurement reagents, glucose assay kits and glucose sensors

## Claims

1. A method for lowering the activity for maltose in glucose measurement which comprises a step of reacting pyrroloquinoline quinone-dependent glucose dehydrogenase, the method comprising a step of keeping the acidic pH during the measurement reaction.

2. The method according to Claim 1, wherein the step of reacting pyrroloquinoline quinone-dependent glucose dehydrogenase is accomplished using a glucose measurement reagent composition comprising pyrroloquinoline quinone-dependent glucose dehydrogenase.

3. The method according to Claim 1, wherein said composition further comprises a mediator.

4. The method according to,Claim 3, wherein the mediator is a ferricyanide salt.

5. The method according to Claim 1, wherein the pyrroloquinoline quinone-dependent glucose dehydrogenase is modified pyrroloquinoline quinone-dependent glucose dehydrogenase the maltose activity of which is lowered below that of the corresponding wild-type enzyme.

6. The method according to Claim 1, wherein the pH is an acidic pH of 6.5 or less during the measurement reaction.

7. The method according to Claim 6, wherein the glucose measurement reagent composition is in a form comprising ferricyanide ions as the mediator.

8. A method for lowering the activity for maltose in glucose measurement comprising a step of reacting the pyrroloquinoline quinone-dependent glucose dehydrogenase according to Claim 6, wherein the glucose measurement composition forms part of a glucose assay kit.

9. The method according to Claim 8, wherein the glucose assay kit comprises pyrroloquinoline quinone-dependent glucose dehydrogenase, and the pH is an acidic pH of 6.5 or less during the measurement reaction.

10. The method according to Claim 1, wherein the step of reacting the pyrroloquinoline quinone-dependent glucose dehydrogenase is accomplished in a glucose sensor including pyrroloquinoline quinone-dependent glucose dehydrogenase and also including electrodes comprising at least a working electrode and a counter electrode.

11. The method according to Claim 10, wherein the reaction in the glucose sensor comprises applying voltage to a reaction solution comprising pyrroloquinoline quinone-dependent glucose dehydrogenase, and measuring the oxidation current of a mediator.

12. The method according to Claim 10, wherein the glucose sensor comprises pyrroloquinoline quinone-dependent glucose dehydrogenase, and wherein the pH is an acidic pH of 6.5 or less during the measurement reaction.

13. The method according to Claim 10, wherein the glucose sensor comprises ferricyanide ions as the mediator.

14. A glucose measurement composition, comprising modified pyrroloquinoline quinone-dependent glucose dehydrogenase having a modification in the amino acid sequence and a buffer capable of maintaining an acidic pH during the measurement reaction, wherein the activity for maltose is reduced.

15. A glucose sensor comprising modified pyrroloquinoline quinone-dependent glucose dehydrogenase having mutations in the amino acid sequence and a buffer capable of maintaining an acidic pH during the measurement reaction, wherein the activity for maltose is reduced.
